(19) Europäisches Patentamt
European Patent Office
Office européen des brevets

(11) Veröffentlichungsnummer: **0 249 603**
**B1**

(12) # EUROPÄISCHE PATENTSCHRIFT

(45) Veröffentlichungstag der Patentschrifft: **21.03.90**

(51) Int. Cl. [5]: **A 61 L 33/00**

(21) Anmeldenummer: **86906774.4**

(22) Anmeldetag: **18.11.86**

(86) Internationale Anmeldenummer: **PCT/DE 86/00471**

(87) Internationale Veröffentlichungsnummer: **WO 87/03209 (04.06.87 Gazette 87/12)**

(54) **ANTITHROMBOGENES, NICHTCALCIFIZIERENDES MATERIAL UND EIN VERFAHREN ZUR HERSTELLUNG VON GEGENSTÄNDEN FÜR MEDIZINISCHE ZWECKE.**

(30) Priorität: **23.11.85 DE 3541478**

(43) Veröffentlichungstag der Anmeldung: **23.12.87 Patentblatt 87/52**

(45) Bekanntmachung des Hinweises auf die Patenterteilung: **21.03.90 Patentblatt 90/12**

(84) Bennante Vertragsstaaten: **AT BE CH DE FR GB IT LI NL SE**

(56) Entgegenhaltungen:
**WO-A-40/1879**
**US-A-4 507 418**

(73) Patentinhaber: **Beiersdorf Aktiengesellschaft**
**Unnastrasse 48**
**D-2000 Hamburg 20 (DE)**

(72) Erfinder: **PIETSCH, Hanns**
**Mittelweg 25**
**D-2000 Hamburg 13 (DE)**
Erfinder: **SACHAU, Günther**
**Lessingstrasse 21**
**D-2085 Quickborn (DE)**
Erfinder: **KARTHEUS, Holger**
**Stellinger Weg 19**
**D-2000 Hamburg 20 (DE)**
Erfinder: **HOLTZMANN, Hans-Joachim**
**Borchertring 64**
**D-2000 Hamburg 60 (DE)**
Erfinder: **REUL, Helmut**
**Akazienstrasse 65**
**D-5160 Düren (DE)**

**EP 0 249 603 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die Erfindung betrifft ein antithrombogenes, nichtcalcifizierendes und elastisches Material auf Polyurethanbasis und ein Verfahren zur Herstellung von Gegenständen für medizinische Zwecke, die sowohl für den Kurzzeit- als auch den Langzeit-Gebrauch in Kontakt mit menschlichem Blut geeignet sind.

Es ist bekannt, daß Polyurethane sich wegen ihrer mechanischen Eigenschaften wie Elastizität, Flexibilität, Reißfestigkeit und ihrer - bis zu einem gewissen Grade - antithrombogenen Eigenschaft für die Herstellung von medizinischen Gegenständen wie z. B. Herzklappenprothesen, Überzügen an Herzschrittmacher-Elektroden, Blutbeuteln, Kathetern u. dgl. eignen. Es sind auch eine ganze Reihe von Patenten und Patentanmeldungen veröffentlicht worden (beispielsweise DE-PS-1 944 969, DE-OS-2 642 616, DE-OS-3 107 542, DE-OS-3 130 646, DE-OS-3 318 730, DE-OS-3 606 440, EP-PS-68 385, EP-OS-129 396, EP-OS-152 699, EP-OS-184 465, US-PS-4 350 806, US-PS-4 371 686, US-PS-4 600 652), die sich damit befassen, durch Zusätze oder spezielle Auswahl der Komponenten vor allem die Blutverträglichkeit der Polyurethane zu verbessern und zu optimieren.

Inzwischen hat sich jedoch gezeigt, daß die Blutverträglichkeit der Materialien, d.h. in erster Linie ihre antithrombogene Wirkung, nicht der einzige kritische Punkt ist, sondern daß Verkalkungsablagerungen und bei Langzeitanwendung der biologische Abbau mindestens ebenso Probleme bereitet.

Bei den sogenannten Bioprothesen aus natürlichem Gewebe, das vorzugsweise mit Glutaraldehyd vernetzt wurde, ist es bekannt, diese als Maßnahme gegen Verkalkung beispielsweise mit einer wäßrigen Lösung von Alkylsulfaten (EP-PS-65 827) oder von wasserlöslichen Salzen von Alkylphosphorsäureestern wie Natriumdodecylhydrogenphosphat (US-PS-4 402 697) zu waschen. Diese Verfahren lassen sich jedoch nicht auf Implantate wie Herzklappenprothesen aus Polyurethan übertragen, da solche Salze nicht auf Polyurethane aufziehen.

Aufgabe der Erfindung war es deshalb, an sich bekannte Polyurethane, seien es Polyetherurethane, Polyesterurethane, Polyetherurethanharnstoffe oder Polyesterurethanharnstoffe oder deren bekannte Gemische bzw. Mischpolymerisate mit modifizierenden Verbindungen, in der Weise weiter zu verbessern, daß sie außerdem nicht-calcifizierend wirken. D.h., daß auch bei längerer Verweildauer im Körper sich an daraus gefertigten Produkten keine oder so gut wie keine Verkalkungen ablagern.

Überraschenderweise wurde gefunden, daß diese Aufgabe durch einen Zusatz von Fettsäureestern zu den Polyurethanen gelöst wird und zwar vorzugsweise durch Fettsäureester aus aliphatischen, unverzweigten, gesättigten oder einfach oder zweifach ungesättigten Monocarbonsäuren mit 10 - 20 C-Atomen und ein- oder mehrwertigen aliphatischen Alkoholen mit 1 - 6 C-Atomen oder Ascorbinsäure, oder auch durch Polyoxyethylenfettsäureester und Polyoxyethylensorbitanfettsäureester.

Bei den Fettsäuren handelt es sich in erster Linie um Säuren wie Laurinsäure, Ölsäure, Palmitinsäure und Myristinsäure, bei den Alkoholen um Verbindungen wie Methanol, Ethanol, Propanol, Isopropanol, Butanol-1, Butanol-2, Isobutanol-1, Isobutanol-2, tert.-Butanol, Ethandiol, Propandiol-1,2, Propandiol-1,3, Ascorbinsäure, Glyzerin oder Sorbitan. Wobei die beiden letzteren auch zwei- und dreifach verestert sein können.

Von den Polyoxyethylenfettsäureestern und Polyoxyethylensorbitanfettsäureestern eignen sich unter anderen besonders Polyoxyethylenmonostearat und Polyoxyethylensorbitanmonooleat.

Die Ester müssen mindestens eine Fettsäuregruppe aufweisen und werden zweckmäßig so ausgewählt, daß sie mit dem vorgesehenen Polyurethan verträglich sind, d.h. als flüssige Ester keine Trübung ergeben. Außerdem sollten die Ester eine gute histologische Verträglichkeit aufweisen. Diese ist umso besser, je geringer die Wasserlöslichkeit ist.

Zu den bevorzugten Estern zählen auch solche Verbindungen, die bereits als Hilfsstoffe für Arzneimittel zugelassen und üblich sind, wie Isopropyl-myristat und -palmitat, Ascorbylpalmitat, Sorbitanmono-laurat, -oleat, -palmitat und -stearat.

Die Ester werden in Mengen von 0,1 - 10 Gew.-%, vorzugsweise 0,5 - 5 Gew.-%, bezogen auf das Gesamtgewicht, der Mischung zugefügt, um eine gute anticalcifizierende Wirkung zu erhalten.

Bei den in Frage kommenden Polyurethanen handelt es sich in erster Linie um lineare und thermoplastische und gegebenenfalls in organischen Lösungsmitteln lösliche Verbindungen, wobei die chemische Zusammensetzung des Polyurethans maßgebend für die Art des Lösungsmittels ist. Beispiele für häufig geeignete Lösungsmittel sind N,N-Dimethylformamid oder N,N-Dimethylacetamid oder deren Mischungen untereinander oder deren Mischungen mit anderen organischen Lösungsmitteln.

Vorzugsweise werden lineare, segmentierte Polyetherurethanharnstoffe und Polyesterurethanharnstoffe eingesetzt. Diese enthalten neben den Urethangruppen auch Harnstoffgruppen.

Ihre Herstellung erfolgt in an sich bekannter Weise derart, daß Polyether oder Polyester mit endständigen Hydroxylgruppen mit Di-isocyanat-Verbindungen zu Präpolymeren mit endständigen Isocyanatgruppen umgesetzt werden und diese sodann mit geeigneten Diaminoverbindungen zu hochmolekularen Polyether- bzw. Polyesterurethanharnstoffen umgesetzt werden.

Für die das Weichsegment bildenden Polyether eignen sich bevorzugt Polyethylenglycol, Polypropylenglycol-(1,2), Polypropylenglycol-(1,3), Polyethylenglycol-Polypropylenglycol-(1,2)-Copolymerisat und Polybutylenglycol-(1,4) oder deren Gemische, wobei die Polyether eine mittleres Molgewicht von 500 - 20 000, vorzugsweise 800 - 5 000, aufweisen sollten.

Für die das Weichsegment bildenden Polyester eignen sich bevorzugt Verbindungen aus aliphatischen Dicarbonsäuren und aliphatischen Diolen mit 2 - 10 Methylengruppen, wie beispielsweise aus den Säuren Malonsäure, Bernsteinsäure, Glutarsäure, Adipinsäure, Pimelinsäure, Korksäure, Azelainsäure, Sebacinsäure oder auch Terephthalsäure mit den Alkoholen Glycol, Diglycol, 1,2-Dihydroxypropan, 1,3-Dihydroxypropan, 1,4-Dihydroxybutan,

1,5-Dihydroxypentan, 1,6-Dihydroxyhexan, 1,7-Dihydroxyheptan, 1,8-Dihydroxyoctan, 1,9-Dihydroxynonan oder 1,10-Dihydroxydecan.

Die Polyester sollten ein mittleres Molgewicht von 500 - 20 000, vorzugsweise von 800 - 10 000 aufweisen.

Als Diisocyanat- und Diaminokomponenten werden bevorzugt solche Verbindungen verwendet, die sich vom Diphenylmethan ableiten, d.h. zwischen den Phenylkernen eine aktive Methylengruppe enthalten, wie beispielsweise 4,4'-Diisocyanatodiphenylmethan oder 4,4'-Diaminodiphenylmethan. Jedoch lassen sich auch andere Diisocyanate einsetzen, wie 1,6-Hexamethylendiisocyanat, 2,4-Toluylendiisocyanat, 2,6-Toluylendiisocyanat, 1,4-Phenylendiisocyanat, 1,2-Phenylendiisocyanat, 4,4'-Dicyclohexylmethandiisocyanat, Isophorondiisocyanat, Methylcyclohexyldiisocyanat, p-Xylylendiisocyanat, 1-Chlor-2,4-phenylendiisocyanat, Trimethylhexamethylendiisocyanat, 4,4'-Diisocyanato-3,3'-dimethyldiphenylmethan oder 4,4'-Diisocyanato-3,3'-dimethylbiphenyl.

Als weitere geeignete Diamino-Verbindungen seien beispielsweise Diaminobenzol, Diaminotoluol oder p,p'-Isopropylidendi(phenylamin) genannt.

Ein weiterer Nachteil bisher verwendeter Polyurethane ist, besonders bei Langzeitanwendung der daraus hergestellten Produkte, der biologische Abbau durch Hydrolyse und enzymatischen Abbau. Die Esterbindungen von Polyesterurethanen können durch Esterasen, die Urethanbindungen durch Proteasen und die Harnstoffbindungen durch Ureasen gespalten werden. Derartige Abbauerscheinungen, die an verschiedenen blutverträglichen Polyurethanen untersucht wurden, erfolgen je nach der Hydrophilie des jeweiligen Polyurethans etwas unterschiedlich schnell, setzen vorzugsweise an der Oberfläche ein und führen dazu, daß die zunächst glatte Oberfläche erodiert und gefurcht wird. Diese Oberflächenrauhigkeiten bilden dann die mechanischen Ansatzpunkte für Blut- und Kalkablagerungen. Außerdem wird durch den Abbau auch die mechanische Stabilität des Produkts geschwächt.

Es war deshalb eine weitere Aufgabe der Erfindung, diese Tendenz zum Abbau zu verhindern. Dieses Problem wird durch den Einsatz vernetzter Polyurethane gelöst, wobei erfindungsgemäß vorteilhafterweise so vorgegangen wird, daß die Formgebung noch mit unvernetztem Material aus der Lösung oder thermoplastisch, beispielsweise im Spritzgußverfahren, durchgeführt wird und die Vernetzung erst nachträglich am geformten Produkt durch die Einwirkung von Wasser oder Luftfeuchtigkeit erfolgt. Durch die Vernetzung wird das Urethan unlöslich in den Lösungsmitteln, in denen es vorher löslich war.

Die Vernetzung erfolgt bevorzugt mit Hilfe von Alkoxysilyl-Verbindungen, die mindestens zwei Methoxy-, Ethoxy-, Propoxy- oder Butoxysilylgruppen und mindestens eine Aminogruppe enthalten, vorzugsweise mit γ-Aminopropyltrisethoxysilan. Weitere Vernetzer sind z. B. N-Aminoethyl-aminopropyltrimethoxysilan oder sogenanntes "Triaminosilan"

($H_2N$-$CH_2$-$CH_2$-NH-$CH_2$-$CH_2$-NH-$CH_2$-$CH_2$-$CH_2$-Si(O)$_3$ mir R = -$CH_3$ oder -$C_2H_5$).

Die Vernetzer sollten, je nach Polyurethan und gewünschten Eigenschaften des Endproduktes, in Mengen von 0,1 bis etwa 5 Gew.-% bezogen auf das Polyurethan zugegeben werden, um eine ausreichende Abbauresistenz zu bewirken.

Bevorzugte Mischungen enthalten 85 - 99,9 Gew.-% der aufgeführten Polyurethane, 0,1 - 10 Gew.-% der genannten Fettsäureester und gegebenenfalls bis zu 5 Gew.-% Vernetzer, jeweils bezogen auf das Gesamtgewicht der Mischung.

Erfindungsgemäß wurde außerdem ein Verfahren zur Herstellung von Gegenständen für medizinische Zwecke unter Verwendung des neuen Materials entwickelt, wobei besonders große Sorgfalt auf die Reinigung des Materials gelegt wurde, um toxische Monomere, Oligomere oder Hilfsstoffe zu entfernen.

Dieses Verfahren besteht im wesentlichen aus folgenden Schritten:

Nachdem die Polyurethane nach bekannten Verfahren (beispielsweise gemäß US-PS-2 929 804) entweder in Lösung oder lösungsmittelfrei hergestellt worden sind, werden aus diesen die restlichen Monomeren, Oligomeren und Katalysatoren herausextrahiert. Als Extraktionsmittel dienen organische Lösungsmittel in denen letztere löslich sind, nicht jedoch das Polymere. Solche Lösungsmittel sind beispielsweise Methanol, Ethanol, Isopropanol, Aceton, Butanon, Hexan, n-Octan, iso-Octan, Cyclohexan, Benzol, Toluol, Ameisensäure-methyl-, -ethyl-, -isopropyl-ester, Essigsäure-methyl-, -ethyl-, -isopropyl-ester, Propionsäure-methyl-, -ethyl-, -isopropyl-ester, vorzugsweise Essigsäureethylester.

Das Waschen erfolgt erfindungsgemäß durch Extraktion bei Raumtemperatur unterhalb von 30°C. Anschließend wird anhaftendes Lösungsmittel durch Trocknen unterhalb 30°C entfernt. Soll die Verarbeitung durch Tauchlackierung (Tauchbeschichtung) erfolgen, wird das gereinigte Polyurethan in einem geeigneten Lösungsmittel gelöst. Das Lösen soll unter möglichst geringer Temperaturbelastung vorzugsweise unterhalb von 30°C durchgeführt werden, was jedoch nicht immer möglich ist. In eine solche Lösung kann der Fettsäureester und gegebenenfalls der Vernetzer, die jeweils beide flüssig sind, eingearbeitet werden.

Soll das Material thermoplastisch verarbeitet werden, lassen sich Fettsäureester und gegebenenfalls Vernetzer mit dem festen Polyurethan vermischen. Je nachdem, in welcher Form das Polyurethan vorliegt, als Pulver, Pellet, Granulat oder Faser, können Fettsäureester und gegebenenfalls Vernetzer entweder in einer Mischtrommel oder durch eine entsprechende Dosiereinrichtung zu dem Urethan zugefügt werden.

Da die erfindungsgemäßen Vernetzer wasser- bzw. feuchtigkeitsempfindlich sind, muß die Mischung vor der Verarbeitung gegen vorzeitige Vernetzung vor Feuchtigkeit geschützt gelagert werden.

Die Weiterverarbeitung kann entweder aus Lösung oder lösungsmittelfrei unter Ausnutzung der thermoplastischen Eigenschaften des Materials geschehen. Beim Lösungsmittelverfahren wird die Form des zukünftigen Produktes, z. B. die Segelklappen einer Herzklappenprothese, ein- oder vorzugsweise mehrmalig in die Lösung des Polyurethans und der Zusatzstoffe getaucht und dazwischen jeweils weitgehend getrocknet bei Temperaturen zwischen 30 - 60°C und einer relativen Luftfeuchtigkeit von 20 %., wobei die Form in ständiger Rotation gehalten wird, um eine gleichmäßige Schichtdicke zu erzielen. Danach erfolgt die vollständige Trocknung und anschließende Behandlung mit entionisiertem, gegebenenfalls keimfreien Wasser. Diese Behandlung entzieht dem Gegenstand restliche, wasserextrahierbare Substanzen und ermöglicht die Vernetzung, wenn ein Vernetzer vorhanden ist.

Erfolgt die Verarbeitung thermoplastisch, z. B. in einer Spritzgußmaschine, einem Extruder oder Kalander oder einer Tiefziehmaschine, werden die Gegenstände, nachdem sie ihre endgültige Form erreicht haben, auf die gleiche Weise mit Wasser behandelt.

Bei Verwendung der erfindungsgemäßen Mischungen und bei Einhaltung der genannten Verfahrensweisen erhält man hervorragend blutverträgliche, nichtcalcifizierende Gegenstände. Die vernetzte Variante ist außerdem insbesondere für den Dauereinsatz, z. B. in flexiblen Herzklappenprothesen, in Blutpumpen ("Kunstherz"), als Membran, als Ummantelung von Herzschrittmacher-Elektroden, für angiographische oder Herzkatheter, als künstliches Pericard, als Material für Schläuche und Ventile oder Beutel, die mit Blut in Kontakt kommen, geeignet.

Die guten mechanischen Eigenschaften der Polyurethane wurden durch die erfindungsgemäßen Zusätze nicht verändert. Deswegen werden diese in den nachfolgenden Beispielen nicht erwähnt, wohl aber die biochemischen Eigenschaften. Zur Prüfung der allgemeinen Zell- und Gewebeverträglichkeit wurden die Materialien mit 3T3-Zellkulturen geprüft. Die Hämolyse wurde nach ASTM F 756 bestimmt. Die Blutverträglichkeit wurde in-vitro geprüft nach: J. P. Fischer, P. Fuhge, K. Burg, H. Heimburger, "Methoden zur Herstellung und Charakterisierung von Kunststoffen mit verbesserter Blutverträglichkeit", Angew. Makromol. Chem. 105 (1982), 131 - 165.

Die anticalcifizierenden Eigenschaften wurden geprüft nach: B. Glasmacher, H. Reul, G. Rau, C. Erckes, J. Wieland "In vitro investigation of the calcification behaviour of polyurethane biomaterials"; Vortrag, gehalten auf dem internationalen Congress "Polyurethanes in biomedical engineering"; 18. - 19.06.1986.

Beide in-vitro-Tests berücksichtigen die komplexe Natur des Blutgerinnungsverhaltens sowie der Calcifizierung.

Die Erfindung wird anhand der nachfolgenden Beispiele näher erläutert.

**Beispiel 1**

1100 g thermoplastischer Polyetherurethanharnstoff (Lycra T137C, Fa. Du Pont) in Form von Fäden wurden mit 7 l Essigsäureethylester versetzt und 24 Stunden bei 15 - 25°C extrahiert und sodann bei Raumtemperatur getrocknet. Der Gewichtsverlust betrug ca. 8 - 11 %. Die extrahierten und getrockneten Fäden wurden bei Raumtemperatur in Dimethylacetamid zu einer 10-prozentigen Lösung aufgelöst. Diese Lösung diente als Stammlösung PU-1 für die nachfolgenden Beispiele.

**Beispiele 2 - 5**

Die gemäß Beispiel 1 hergestellte Lösung (Stamm-PU 1) wurde mit Fettsäureestern versetzt.

| Beispiel | Stamm-PU 1 (Gew.-%) | Fettsäureester (Gew.-%) |
|---|---|---|
| 2 | 97 % | 3,0 % Palmitinsäureisopropylester |
| 3 | 95 % | 5,0 % Laurinsäurebutylester |
| 4 | 99,5 % | 0,5 % Sorbitantrioleat |
| 5 | 97 % | 3,0 % Polyoxyethylenmonostearat |

Aus den Fettsäureester-haltigen Lösungen wurden unter staubfreien Bedingungen in einem Reinraum Gießfolien in einer Dicke von 0,3 mm hergestellt. Alle Folien waren klar und besaßen glatte Oberflächen. Ihre Blutverträglichkeit wurde entsprechend Fischer et. al. geprüft und ergab in allen Fällen eine Verträglichkeit die besser war als die des Stammpolymerisats aus Beispiel 1.

Die Verkalkung wurde entsprechend Glasmacher et. al. geprüft und ergab in allen Fällen günstigere Werte als die des Stammpolymerisats aus Beispiel 1.

**Beispiele 6 - 8**

Die gemäß Beispiel 1 hergestellte Stammpolymer-Lösung wurde mit Fettsäureestern und Vernetzern versetzt.

| Beisp. | Stamm-PU (Gew.-%) | Fettsäureester (Gew.-%) | Vernetzer (Gew.-%) |
|---|---|---|---|
| 6 | 96 % | 3 % Palmitinsäureisopropylester | 1 % γ-Aminopropyltrisethoxysilan |
| 7 | 96,5 % | 3 % Polyoxyethylensorbitanmonooleat | 0,5 % N-Aminoethyl-Aminopropyltrimethoxysilan |
| 8 | 93 % | 5 % Laurinsäureethylester | 2 % "Triaminosilan" *) |

*) $H_2N-CH_2-CH_2-NH-CH_2-CH_2-NH-CH_2-CH_2-CH_2-Si(OCH_3)_3$

Aus diesen Lösungen wurden unter staubfreien Bedingungen in einem Reinraum wiederum Gießfolien hergestellt. Die Gießfolien wurden nach Abdunsten des Lösungsmittels 24 Stunden in entionisiertes Wasser bei 60°C gebracht, anschließend getrocknet und dann zur Prüfung auf Vernetzung mit Dimethylacetamid versetzt und 24 Stunden auf einer Schüttelmaschine geschüttelt. Alle Folien waren unlöslich. Die Folien entsprechend den Beispielen 2 - 5 waren beim gleichen Versuch dagegen löslich.

Die Prüfung der Folien auf Blutverträglichkeit und Verkalkungsneigung ergab im Vergleich wiederum bessere Werte als für eine Folie aus dem Ursprungsmaterial des Beispiel 1.

**Beispiel 9**

1100 g Polyetherurethan (Pellethane 2363 - 80A) in Form von Granulat wurden mit 7 l Essigsäureethylester versetzt und 24 Stunden bei 15 - 25°C extrahiert und getrocknet. Der Gewichtsverlust betrug 1 - 2 %. Das extrahierte und getrocknete Polyurethan wurde mit einer Mischung aus Dimethylformamid und Xylol (o-/p-Gemisch in einem Gewichtsverhältnis von 1 : 1) während 30 Stunden bei 100°C gelöst. Die Lösung enthielt weniger als 1 % unlösliche Anteile, die abfiltriert wurden.

Diese klare, leicht gelbstichige Lösung wurde für weitere Versuche als Stammlösung PU-2 eingesetzt.

**Beispiele 10 - 16**

Die nach Beispiel 9 hergestellte Lösung wurde mit Fettsäureestern versetzt.

| Beispiel | Stamm-PU-2 (Gew.-%) | Fettsäureester (Gew.-%) |
|---|---|---|
| 10 | 96 % | 4 % Laurinsäureethylester |
| 11 | 90 % | 10 % Sorbitanmonooleat |
| 12 | 92 % | 8 % Ascorbylpalmitat |
| 13 | 99 % | 1 % Myristinsäureisopropylester |
| 14 | 97 % | 3 % Palmitinsäureisopropylester |
| 15 | 95 % | 5 % Laurinsäurebutylester |
| 16 | 99,5 % | 0,5 % Polyoxyethylensorbitanmonooleat |

Aus diesen Lösungen wurden unter staubfreien Bedingungen in einem Reinraum Gießfolien hergestellt. Messungen der Blutverträglichkeit und der Verkalkungen ergaben wieder eine Verbesserung der Eigenschaften gegenüber dem undotierten Stamm-PU 2.

**Beispiel 17**

1100 g Polyesterurethan (Estane 58 206) in Form von Granulat wurden mit 7 l Essigsäureethylester versetzt, 24 Stunden extrahiert und getrocknet. Das extrahierte und getrocknete Polyurethan wurde in einem Extruder zu einem Strang verarbeitet, der 2 mm dick und 2 cm breit war (Stamm-PU 3). Aus diesem Strang wurden Probestücke zur Messung der Blutverträglichkeit und der Calcifizierungstendenz geschnitten.

**Beispiele 18 - 21**

Aus dem nach Beispiel 17 gereinigten Polyesterurethan wurden im Extruder verschiedene Mischungen mit Fettsäureestern hergestellt.

| Beispiel | Stamm-PU 3 (Gew.-%) | Fettsäureester (Gew.-%) |
|---|---|---|
| 18 | 97 % | 3 % Palmitinsäureisopropylester |
| 19 | 97 % | 3 % Laurinsäurebutylester |
| 20 | 97 % | 3 % Myristinsäureisopropylester |
| 21 | 97 % | 3 % Ascorbylpalmitat |

Die Mischungen wurden zu Strängen gepreßt und aus diesen die Teststücke für Blutverträglichkeit und Calcifizierung entnommen. Auch in diesem Fall erwiesen sich die Eigenschaften der Materialien gemäß Beispiel 18 bis 21 dem unvermischten Material überlegen.

**Patentansprüche**

1. Antithrombogenes, nichtcalzifizierendes, elastisches Material auf Polyurethanbasis, dadurch gekennzeichnet, daß es einzeln oder im Gemisch 0,1 - 10 Gew.-%, vorzugsweise 0,5 - 5 Gew.-% bezogen auf das Gesamtgewicht, Fettsäureester aus aliphatischen, unverzweigten, gesättigten oder einfach oder zweifach ungesättigten Monocarbonsäuren mit 10 - 20 C-Atomen und ein- oder mehrwertigen aliphatischen Alkoholen mit 1 - 6 C-Atomen oder Ascorbinsäure, Polyoxyethylenfettsäureester oder Polyoxyethylensorbitanfettsäureester sowie als Vernetzer einzeln oder im Gemisch 0,1 - 5 Gew.-%, bezogen auf das Gesamtgewicht, Alkoxysilyl-Verbindungen, die mindestens zwei Methoxy-, Ethoxy-, Propoxy- oder Butoxysilylgruppen und mindestens eine Aminogruppe aufweisen, enthält.

2. Verwendung von elastischen Polyurethanen, die einzeln oder im Gemisch 0,1 - 10 Gew.-%, vorzugsweise 0,5 - 5 Gew.-% bezogen auf das Gesamtgewicht, Fettsäureester aus aliphatischen, unverzweigten, gesättigten oder einfach oder zweifach ungesättigten Monocarbonsäuren mit 10 - 20 C-Atomen und ein- oder mehrwertigen aliphatischen Alkoholen mit 1 - 6 C-Atomen oder Ascorbinsäure, Polyoxyethylenfettsäureester oder Polyoxyethylensorbitanfettsäureester sowie gegebenenfalls als Vernetzer einzeln oder im Gemisch 0,1 - 5 Gew.-%, bezogen auf das Gesamtgewicht, Alkoxysilyl-Verbindungen, die mindestens zwei Methoxy-, Ethoxy-, Propoxy- oder Butoxysilylgruppen und mindestens eine Aminogruppe aufweisen, enthalten, als antithrombogenes, nichtcalzifizierendes Material.

3. Material gemäß Anspruch 1 oder 2, dadurch gekennzeichnet, daß das Polyurethan ein linearer, segmentierter Polyetherurethanharnstoff oder ein linearer, segmentierter Polyesterurethanharnstoff ist.

4. Verfahren zur Herstellung von Gegenständen für medizinische Zwecke aus einem antithrombogenen, nichtcalzifizierenden Material gemäß Anspruch 1 oder 2, dadurch gekennzeichnet, daß die Herstellung in folgendem Ablauf erfolgt:

1. Herstellung des Polyurethans,
2. Waschen und Trocknen des Polyurethans mit organischen Lösungsmitteln, in denen das Polyurethan nicht löslich ist,
3. Lösen des Polyurethans, wenn aus Lösung verarbeitet werden soll,
4. Zugabe der Fettsäureester und gegebenenfalls des Vernetzungsmittels zum trockenen Polyurethan oder zu seiner Lösung,
5. Herstellung der Gegenstände,
6. Waschen der Gegenstände mit Wasser, wobei gleichzeitig gegebenenfalls die Vernetzung stattfindet,
7. Endtrocknung der Gegenstände.

5. Verfahren gemäß Anspruch 4, dadurch gekennzeichnet, daß das Waschen, Lösen und Trocknen des Polyurethans unterhalb 30°C durchgeführt wird.

**Claims**

1. Antithrombogenic, non-calcifying elastic material based on polyurethane, characterized in that it contains, individually or in the mixture, 0.1 - 10 % by weight, preferably 0.5 - 5 % by weight, relative to the total weight, of fatty acid esters of aliphatic, unbranched, saturated or mono- or diunsaturated monocarboxylic acids having 10 - 20 C atoms and mono- or polyhydric aliphatic alcohols having 1 - 6 C atoms, or ascorbic acid, polyoxyethylene fatty acid esters or polyoxyethylene sorbitan fatty acid esters and, as the crosslinker, individually or in the mixture, 0.1 - 5 % by weight, relative to the total weight, of alkoxysilyl compounds which have at least two methoxy-, ethoxy-, propoxy- or butoxysilyl groups and at least one amino group.

2. Use of elastic polyurethanes which contain, individually or in the mixture, 0.1 - 10 % by weight, preferably 0.5 - 5 % by weight, relative to the total weight, of fatty acid esters of aliphatic, unbranched, saturated or mono- or diunsaturated monocarboxylic acids having 10 - 20 C atoms and mono- or polyhydric aliphatic alcohols having 1 - 6 C atoms, or ascorbic acid, polyoxyethylene fatty acid esters or polyoxyethylene sorbitan fatty acid esters and if appropriate, as the crosslinker, individually or in the mixture, 0.1 - 5 % by weight, relative to the total weight, of alkoxysilyl compounds which have at least two methoxy-, ethoxy-, propoxy- or butoxysilyl groups and at least one amino group, as antithrombogenic, non-calcifying material.

3. Material according to claim 1 or 2, characterized in that the polyurethane is a linear, segmented polyetherurethane urea or a linear, segmented polyesterurethane urea.

4. Method for the production of articles for medical purposes from an antithrombogenic, non-calcifying material according to claim 1 or 2, characterized in that the production is carried out in the following sequence:

1. preparation of the polyurethane,
2. washing and drying of the polyurethane with organic solvents in which the polyurethane is not soluble,
3. dissolving the polyurethane if it is intended to be processed from solution,

4. addition of the fatty acid ester and, if appropriate, the crosslinking agent to the dry polyurethane or to its solution,
5. production of the articles,
6. washing the articles with water, if appropriate the crosslinking taking place simultaneously,
7. final drying of the articles.

5. Process according to claim 4, characterized in that the washing, dissolving and drying of the polyurethane is carried out below 30°C.

**Revendications**

1. Matière élastique, antithrombogène, non calcifiante à base de polyuréthanne, caractérisée en ce qu'elle contient, isolément ou en mélange, 0,1 - 10 % en poids, de préférence 0,5 - 5 % en poids, sur la base du poids total, d'esters d'acides gras issus d'acides monocarboxyliques aliphatiques non ramifiés saturés ou une ou deux fois insaturés comptant 10 - 20 atomes de carbone et d'alcools aliphatiques mono- ou polyvalents comptant 1 - 6 atomes de carbone ou d'acide ascorbique, d'esters d'acides gras de polyoxyéthylène ou d'esters d'acides gras de polyoxyéthylènesorbitan, ainsi que comme agents de réticulation, isolément ou en mélange, 0,1 - 5 % en poids, sur la base du poids total, d'alcoxysilyl-composés qui contiennent au moins deux radicaux méthoxy-, éthoxy-, propoxy- ou butoxy-silyle et au moins un radical amino.

2. Utilisation de polyuréthannes élastiques qui contiennent, isolément ou en mélange, 0,1 - 10 % en poids, de préférence 0,5 - 5 % en poids, sur la base du poids total, d'esters d'acides gras issus d'acides monocarboxyliques aliphatiques non ramifiés saturés ou une ou deux fois insaturés comptant 10 - 20 atomes de carbone et d'alcools aliphatiques mono- ou polyvalents comptant 1 - 6 atomes de carbone ou d'acide ascorbique, d'esters d'acides gras de polyoxyéthylène ou d'esters d'acides gras de polyoxyéthylènesorbitan, ainsi que eventuellement comme agents de réticulation, isolément ou en mélange, 0,1 - 5 % en poids, sur la base du poids total, d'alcoxysilyl-composés qui contiennent au moins deux radicaux méthoxy-, éthoxy-, propoxy- ou butoxy-silyle et au moins un radical amino, comme matières antithrombogènes non calcifiantes.

3. Matière suivant la revendication 1 ou 2, caractérisée en ce que le polyuréthanne est une polyétheruréthanne-urée linéaire segmentée ou une polyesteruréthanne-urée linéaire segmentée.

4. Procédé de fabrication d'objets à des fins médicales à partir d'une matière antithrombogène non calcifiante suivant la revendication 1 ou 2, caractérisé en ce que la fabrication a lieu dans l'ordre suivant:

1. fabrication du polyuréthanne,
2. lavage et séchage du polyuréthanne avec des solvants organiques dans lesquels le polyuréthanne n'est pas soluble,
3. dissolution du polyuréthanne, lorsqu'il doit être mis en oeuvre à partir d'une solution,
4. addition des esters d'acides gras et éventuellement de l'agent de réticulation au polyuréthanne sec ou à sa solution,
5. fabrication des objets,
6. lavage des objets avec de l'eau, auquel cas la réticulation a éventuellement lieu en même temps,
7. séchage final des objets.

5. Procédé suivant la revendication 4, caractérisé en ce que le lavage, la dissolution et le séchage du polyuréthanne sont exécutés au-dessous de 30°C.